# EUROPÄISCHE PATENTANMELDUNG

(11) **EP 2 816 104 A1**
(43) Veröffentlichungstag der Anmeldung: **24.12.2014**
(21) Anmeldenummer: 14002902.6
(22) Anmeldetag: 10.11.2004
(51) Int. Cl.: C12N 1/10, C12P 7/64, A23K 1/16, C12N 1/12, C12N 1/14

(54) **Verfahren zur Kultivierung von Mikroorganismen der Gattung Thraustochytriales unter Verwendung eines optimierten Niedrigsalzmediums**

(30) Priorität: 10.11.2003 DE 10352838
(62) Teilanmeldung aus: 04818134.1
(71) Anmelder: Lonza Ltd, 4002 Basel (CH)
(72) Erfinder: Rüsing, Matthias, 50935 Köln (DE); Luy, Markus, 3911 Ried-Brig (CH)
(74) Vertreter: Mai, Dörr, Besier

(57) **Zusammenfassung**

Die Erfindung beschreibt ein optimiertes Verfahren zur Kultivierung von Mikroorganismen der Gattung *Thraustochytriales,* wobei die Mikroorganismen in einem Niedrigsalzmedium ohne Zusatz von Natriumsalzen und Chloridsalzen, bei einem Gesamtsalzgehalt von weniger als 3,5g/L Gesamtsalze (entsprechend weniger als 10% Meerwassersalzgehalt) kultiviert werden und anschließende Isolation der PUFAs aus den Mikroorganismen und/oder dem Medium. Insbesondere werden neue optimierte Medien mit erheblich reduziertem Gesamtsalzgehalt bei besonders reduziertem NaCl-Gehalt beschrieben. Eine neue Kombination verschiedener Salze als Medienzusammensetzung mit in der Summe der Gewichtsanteile nicht mehr als 1,75 g/L Na⁺- und Cl⁻-Ionen erlaubt die Produktion der PUFAs maßgeblich zu verbessern und erheblich zu vereinfachen. Darüber hinaus ist das beschriebene Medium bevorzugt gänzlich frei von Natrium- und Chloridsalz-Zusätzen, was Umweltschäden durch salzhaltige Abwässer zu vermeiden hilft.

## Beschreibung

Verschiedene mehrfach-ungesättigte Fettsäuren (PUFAs für polyunsaturated fatty acids) und insbesondere omega-3 Fettsäuren (n3-Fettsäuren) sind essentielle Bestandteile der menschlichen Ernährung.

Allerdings ist bekannt, dass in den meisten Industrienationen die Versorgung mit n3-Fettsäuren mangelhaft ist. Dagegen ist der Gesamtfettanteil in der Ernährung, sowie die Zufuhr an gesättigten Fettsäuren und n6-Fettsäuren zu hoch. Dies beruht auf einer Veränderung unserer Nahrungszusammensetzung, die vor allem in den letzten ca. 150 Jahren stattgefunden hat und die mit dem Auftreten verschiedener chronischer Zivilisationskrankheiten, wie beispielsweise Herzkreislauferkrankungen - der Haupttodesursache in Industrienationen - korreliert wird (Simopoulos, A.P., 1999, Am. J. Clin. Nutr. 70, 560-569). Eine Vielzahl von Studien hat inzwischen gezeigt, dass durch die gezielte Erhöhung der Zufuhr von n3-Fettsäuren, insbesondere von Eicosapentaensäure (EPA) und von Docosahexaensäure (DHA), das Herzkreislaufrisiko signifikant reduziert werden kann (GISSI-Prevenzione Investigators (Gruppo Italiano per lo Studio della Sopravvivenza nell'Infarto miocardico), 1999, Dietary suplementation with n-3 polyunsaturated fatty acids and vitamin E after myocardial infarction: results of the GISSI-pevenzione trial., Lancet 354, 447-455; Burr et al., 1989, Effects of changes in fat, fish, and fibre intake on death and myocardial reinfarction: diet and reinfarction trial (DART). Lancet 2, 757-761). Dementsprechend wird von vielen verschieden Organisationen (WHO, FAO, AHA; ISSFAL, British Nutrition Foundation u.v.a.) empfohlen, die Zufuhr von n3-Fettsäuren signifikant zu erhöhen (Kris-Eherton et al., Fish Consumption, Fish Oil, Omega-3 Fatty Acids, and Cardiovascular Disease. Circulation 2002, 2747-2757).

Quellen für die Gewinnung von PUFAs und insbesondere n3-Fettsäuren sind vor allem marine Kaltwasserfische und daraus gewonnene Öle, aber auch marine Mikroorganismen, welche gegenüber Fischen den Vorteil haben, dass sie in Fermentern unter kostengünstigen und kontrollierten Bedingungen zur Produktion von PUFAs herangezogen werden können. Bei einer fermentativen Herstellung besteht keine Gefahr der Verunreinigungen, wie sie für Fische oder daraus gewonnene Fischöle oft beschrieben werden (Olsen SF. Int J Epidemiol. 2001:1279-80). Außerdem lässt sich die Zusammensetzung der gewonnenen Öle durch Auswahl des Organismus und der Kulturbedingungen positiv beeinflussen und ist nicht saisonalen Schwankungen unterworfen, wie dies ebenfalls für Fisch und Fischprodukte beschrieben ist (Gamez-Meza et al. Lipids 1999:639-42).

Mikroorganismen, welche sich zur Gewinnung von n3-PUFA eignen, finden sich beispielsweise bei den Bakterien unter der Gattung *Vibrio* (z. B.: *Vibrio marinus)* oder unter den Dinoflagellaten *(Dinophyta),* dort insbesondere die Gattung *Crypthecodinium,* wie C. *cohnii* oder unter den Stramenopiles, wie die *Pinguiophyceae* wie z.B. *Glossomastix, Phaeomonas, Pinguiochrysis, Pinguiococcus* und *Polydochrysis.* Bevorzugte Mikroorganismen zur fermentativen Herstellung von PUFA gehören zu den *Stramenopiles* (oder *Labyrinthulomycota),* insbesondere zur Ordnung *Thraustochytriales,* (*Thraustchytriidea*) und dort wieder insbesondere zu den Gattungen *Japonochytrium, Schizochytrium, Thraustochytrium, Althornia, Labyrinthuloides, Aplanochytrium* und *Ulkenia.*

Es ist bekannt, dass einige der genannten Mikroorganismen zur industriellen Produktion von Fettsäuren verwendet werden können, entsprechende Verfahren wurden beschrieben. So offenbart die internationale Patentanmeldung WO 91/07498 A1 die Herstellung von PUFAs mit Organismen der Gattungen *Thraustochytrium* und *Schizochytrium.* WO 91/11918 A1 offenbart die Herstellung von PUFAs mit *Crypthecodinium cohnii,* WO 96/33263 A1 und die entsprechende europäische Patentanmeldung EP 0 823 475 A1 beschreiben die Herstellung von PUFAs mit Mikroorganismen der Gattung *Schizochytrium,* während die Patentanmeldung WO 98/03671 die Herstellung von PUFAs mit Mikroorganismen der Gattung *Ulkenia* offenbart.

Der natürliche Lebensraum der beschriebenen Mikroorganismen und insbesondere der *Labyrinthulomycota* sind marine Habitate. Herkömmlicherweise werden diese Mikroorganismen also in entsprechend salzhaltigen Medien kultiviert, wobei der Salzgehalt von Meerwasser für die Zwecke der vorliegenden Erfindung mit 32-35 g/L und einem Anteil von 90-95% an Natrium und Chlorid definiert wird. Typische Medien zur Kultivierung von marinen Mikroorganismen wie *Thraustochytrium* oder *Schizochytrium* basieren auf Seewasser (z.B. ATCC (American Type Culture Collection) 790 By+ Medium [Hefeextrakt 1.0 g, Peptone 1.0 g, D+-Glucose 5.0 g, Seewasser 1 L]). Es ist aber auch bekannt, dass Mikroorganismen der Ordnung *Thraustochytriales* bei sehr niedriger Salinität im Kulturmedium überleben können. Ihr Wachstum wird jedoch unterhalb einer Grenze von 7,5 - 15 g Salz/L, entsprechend einer Salinität von 7,5 - 15‰, als nur sehr gering und ohne Zwischenmaxima im niedrigen Salinitätsbereich beschrieben. Optimale Wachstumsraten werden nur oberhalb der oben angegebenen Salinitätsgrenze erzielt (Fan et al. Botanica Marina 45, 2002, S. 50-57).

Zur kommerziellen Fermentation von euryhalinen Mikroorganismen sind aber auch reduzierte Salzgehalte von etwa 50-60% des Meerwassers beschrieben. Nach Henderson's "Dictionary of biological terms" bezeichnet man marine Organismen, die sich an einen weiten Bereich für den Salzgehalt anpassen können, als euryhalin (Henderson W.D., Lawrence, E., Henderson's dictionary of biological terms, 10th ed. 1992 S. 173).

Es wurde beschrieben, dass euryhaline Mikroorganismen der *Stramenopiles* (oder *Labyrinthulomycota*) größere Mengen an PUFA in Fermentationsmedien produzieren können, die einen reduzierten Gehalt an Natrium-Ionen (60% des Meerwassers) enthalten (U.S. Pat. No. 6,451,567). Ebenfalls beschrieben ist die Verwendung von Kulturmedien mit niedrigem Chloridgehalt mit dem Ziel die korrosiven Auswirkungen des Chlorids auf die Fermentationsausrüstung zu reduzieren (U.S. Pat. No 6,410,281). Dies ist beispielsweise für Mikroorganismen der Gattungen *Thraustochytrium* und *Schizochytrium* mit Fermentationsmedien gezeigt worden, welche Chlorid in einer Konzentration von nicht mehr als 3g/L enthalten (U.S. Pat. No 5,340,742, U.S. Pat. No 6,451,567, U.S. Pat. No 6,410,281). Es ist ebenfalls bekannt, dass eine Kultivierung auch bei Bedingungen mit gegenüber Meerwasser reduziertem Salzgehalt möglich ist. Hierbei wird besonders Bezug genommen auf die Patentschriften WO 98/03671 A1, EP 0 823 475 A1, U.S. Pat. No 6,451,567, U.S. Pat. No 6,410,281.

Es ist weiterhin bekannt, dass für die Fermentation eines Mikroorganismus der Gattung *Schizochytrium (Schizochytrium* sp. S31; ATTC 20888) ein Maximum der relativen Ausbeute von Fettsäuren bei einer Natriumchloridkonzentration von 1,75g/L erreicht wird. Die dabei verwendete Gesamtsalzmenge liegt bei weniger als 10% des Meerwassersalzgehaltes, besteht jedoch überwiegend aus Natrium- und Chlorid-Ionen (EP 0 512 997 B1 und U.S. Pat. No 5,518,918)

Alle bisher beschriebenen Verfahren sind jedoch mit Nachteilen behaftet. Die Effektivität von fermentativen Prozessen ist insbesondere durch die erreichbare Biomasse und den Gehalt an Produkt pro Biomasse limitiert. Weiterhin können die gebildeten Öle teilweise Fettsäurespektren aufweisen, die nicht unbedingt den gewünschten Produkten entsprechen, sondern erst verfahrenstechnisch verändert werden müssen. Durch den z.T. geringen Gehalt an Produkt pro Biomasse wird die Aufarbeitung häufig wesentlich verkompliziert, da relativ große Mengen der Biomasse verarbeitet werden müssen, um relativ kleine Mengen an Produkt zu erhalten. Weiterhin sind allen bisher beschriebenen Verfahren relativ hohe Gesamtsalzgehalte in den Kulturmedien zu Eigen. Dies führt nicht nur bei der Aufarbeitung der Produkte zu massiven Problemen, sondern ist umwelttechnisch äußerst nachteilig, da nicht nur große Mengen an Biomasse anfallen, sondern auch sehr salzhaltige Abwässer entstehen, die entsorgt werden müssen.

In Anbetracht des Standes der Technik war es daher Aufgabe der vorliegenden Erfindung ein neues, einfaches und wirtschaftliches Verfahren für die Kultivierung von *Thraustochytriales* zur Verfügung zu stellen, wobei Medien mit verringerten Salzgehalten zur Anwendung kommen sollen. Abgesehen von der Kosteneffizienz sollte das Verfahren einfach durchzuführen sein und die Herstellung hoch reiner PUFAs bzw. von PUFA-enthaltenden Produkten in hoher Ausbeute ermöglichen.

Gelöst werden diese sowie weitere, nicht explizit genannten Aufgaben, die jedoch aus den hierin einleitend diskutierten Zusammenhängen ohne weiteres ableitbar oder erschließbar sind, durch den Gegenstand, der in den Ansprüchen der vorliegenden Erfindung definiert ist.

Ein vorteilhaftes Verfahren für die Kultivierung von *Thraustochytriales* wird durch das in Anspruch 1 definierte Verfahren zur Verfügung gestellt. Dieses Verfahren umfasst das Kultivieren von Mikroorganismen der Ordnung *Thraustochytriales* in einem Niedrigsalzmedium ohne Zusatz von Natrium- oder Chlorid-Ionen in fester oder gelöster Form, bei einem Gesamtsalzgehalt von weniger als 10% bezogen auf Meerwasser, d.h. weniger als ca. 3,5 g/L Gesamtsalze.

Die Erfindung umfasst weiterhin ein Verfahren zur Produktion hochreiner PUFAs.

Bevorzugte PUFAs sind erfindungsgemäß DHA, DPA und EPA.

Insbesondere zeigen die in dem oben genannten Verfahren kultivierten Mikroorganismen eine Produktion von mehr als 10%, bevorzugt mehr als 14%, und ganz besonders bevorzugt mehr als 18% DHA pro Biotrockenmasse.

Insbesondere zeigen die in dem oben genannten Verfahren kultivierten Mikroorganismen eine Produktion von mehr als 5%, bevorzugt mehr als 7%, und ganz besonders bevorzugt mehr als 10% DPA pro Biotrockenmasse.

Durch an die Kultivierung anschließende Isolation der PUFAs aus den Mikroorganismen (Biomasse) und/oder dem Kulturmedium können die PUFAs in hoher Ausbeute und Reinheit gewonnen werden.

Weiterhin umfasst die vorliegende Erfindung auch ein Verfahren zur Herstellung von Biomasse, wobei die Biomasse durch das erfindungsgemäße Kultivierungsverfahren zur Verfügung gestellt wird.

Diese Biomasse kann auf alle erdenklichen Arten Verwendung finden. Insbesondere kann diese Biomasse, z.B. in getrocknetem Zustand (Biotrockenmasse), direkt als Nahrungsmittel oder als Futtermittel eingesetzt werden.

Weiterhin umfasst die Erfindung auch ein Öl, welches dadurch gewonnen wird, dass das erfindungsgemäße Kultivierungsverfahren durchgeführt wird, und das Öl aus den Mikroorganismen (Biomasse) und/oder dem Kulturmedium isoliert wird.

Insbesondere handelt es sich dabei um ein Öl, welches neben vielen weiteren bevorzugten Verwendungszwecken mit Vorteil für die menschliche Ernährung verwendet werden kann.

Dabei zeigen die Mikroorganismen unter erfindungsgemäßen Bedingungen eine Produktion von mehr als 30 Gew.-% Öl, bevorzugt von mehr als 35 Gew.-% Öl pro Gewichtseinheit Biotrockenmasse.

Unter Öl wird erfindungsgemäß ein Anteil von mindestens 70 % Neutrallipiden und mindestens 2% Phospholipiden verstanden, was dem normalen, dem Fachmann bekannten Fettsäurespektrum der *Thraustochytriales* entspricht. Die Neutrallipide bestehen dabei aus mindestens 80% Triglyzeriden und anderen Verbindungen wie z.B Diacylglyzeride, Sterole usw. Weiterhin besteht der Gewichtsanteil der Triglyzeride aus etwa 95% Fettsäuren und 5% Glyzerin. Die Möglichkeit einen marinen Mikroorganismus bei einer solch niedrigen Salzkonzentration, die weniger als 10% des typischen Meersalzgehaltes entspricht, fermentieren zu können, und insbesondere die Tatsache, dass vollständig auf die Zugabe der im Meerwasser dominierenden Ionen Na⁺ und Cl⁻, die normalerweise ca. 90% der im Meerwasser vorhandenen Ionen ausmachen, verzichtet werden kann, war völlig überraschend.

Erstaunlicherweise war nicht nur die Fermentation an sich möglich, sondern es erhöht sich darüber hinaus auch der Anteil der PUFA in der Biomasse bei Einsatz des erfindungsgemäßen Niedrigsalzmediums signifikant. Noch überraschender ist, dass dieser Effekt eine leichte Abnahme der produzierten Biomasse nicht nur ausgleicht, sondern sogar übertrifft (Beispiel 2). Hierbei hat sich der Anteil der überwiegenden PUFA DHA pro Biotrockenmasse gegenüber der Vergleichsfermentation in Medium 1 (50% Meersalzgehalt) um mehr als 10% erhöht. Die Aufarbeitung der Produkte wird durch die höhere Produktkonzentration und die niedrigere Verunreinigung durch Salze vereinfacht und dies bei insgesamt erhöhter Raum-Zeit-Ausbeute.

Bis zur vorliegenden Erfindung gab es keinen bekannten Fermentationsprozess zur Produktion von n3-Fettsäuren in Mikroorganismen aus der Ordnung *Thraustochytriales* unter Verwendung eines Mediums mit derart extrem geringer Salzkonzentration und ohne Natrium-und Chloridsalz- Zusätze.

PUFAs sind erfindungsgemäß mehrfach ungesättigte langkettige Fettsäuren mit einer Kettenlänge >C12 mit mindestens zwei Doppelbindungen. Nach dem erfindungsgemäßen Verfahren herstellbare PUFAs sind insbesondere n3-Fettsäuren und n6-Fettsäuren.

Unter n3-Fettsäuren (omega-3 Fettsäure, ω3 Fettsäuren) im erfindungsgemäßen Sinn werden mehrfach ungesättigte langkettige Fettsäuren mit einer Kettenlänge >C12 mit mindestens zwei oder mehr Doppelbindungen verstanden, wobei die erste der Doppelbindungen zwischen den Kohlenstoffatomen C3 und C4 ausgehend vom Alkylende konstituiert ist. Dementsprechend liegt bei n6-Fettsäuren die erste Doppelbindung zwischen den Kohlenstoffatomen C6 und C7 ausgehend vom Alkylende.

Für die Produktion der PUFAs nach dem erfindungsgemäßen Verfahren kommen Mikroorganismen aus der Gruppe der Labyrinthulomycota in Frage. Mikroorganismen der Ordnung *Thraustochytriales* (*Thraustchytriidea*) sind bevorzugt (Lewis, T.E., Nichols, P.D., McMeekin, T.A., The Biotechnological Potential of Thraustochytrids, Marine Biotechnology, 1999, S. 580-587 und Porter, D. Phylum Labyrinthulomycota in Handbook of protoctista: the structure, cultivation, habitats, and life histories of the eukaryotic microorganisms and their descendants exclusive of animals, plants, and fungi: a guide to the algae, ciliates, foraminifera, sprorozoa, water molds, and other protoctists. Editors: Margulis, L, Corliss, J.O., Melkonian, M. and Chapman, D.J., editorial coordinator, McKhann, H.L, Jones and Bartlett Publishers, ISBN 0-86720-052-9 1990, S. 388-398). Besonders bevorzugt sind Mikroorganismen der Gattungen *Japonochytrium, Labyrinthuloides, Aplanochytrium Althornia, Schizochytrium, Thraustochytrium* und *Ulkenia.* Ganz besonders bevorzugt sind hiervon *Schizochytrium, Thraustochytrium* und *Ulkenia.* Insbesondere bevorzugt sind: *Japonochytrium* sp. ATCC 28207, *Thraustochytrium aureum* (insbesondere ATCC 28211 bzw. ATTC 34304), *Thraustochytrium roseum* ATCC 28210 *Thraustochytrium* sp. ATCC 20890, ATTC 20891, ATTC 20892 und ATTC 26185, *Schizochytrium aggregatum* ATTC 28209, *Schizochytrium* sp. ATCC 20888 und ATTC 20889, *Schizochytrium* SR21, sowie *Ulkenia* spec. SAM 2179 und SAM 2180.

Für das erfindungsgemäße Verfahren geeignete Mikroorganismen sind sowohl Wildtyp Formen als auch Mutanten und daraus abgeleitete Stämme sowie rekombinante Stämme der entsprechenden Organismen. In besonderem Maße umfasst die vorliegende Erfindung Mutanten oder rekombinante Stämme zur Produktionssteigerung von PUFA.

Die Mikroorganismen im Sinne der vorliegenden Erfindung werden durch Animpfen eines flüssigen oder eines festen Mediums mit einer Vorkultur dieser Organismen kultiviert.

Für Mikroorganismen der Ordnung *Thraustochytriales* geeignete Kulturtechniken sind dem Fachmann gut bekannt. Typischerweise, aber nicht ausschließlich, wird die Kultur mittels wässriger Fermentation in einem entsprechenden Behältnis durchgeführt. Beispiele für ein typisches Behältnis für eine derartige Fermentation umfassen Schüttelkolben oder Bioreaktoren, wie beispielsweise STRs (stirred tank reactors) oder Blasensäulen. Die Kultur wird typischerweise bei Temperaturen zwischen 10°C und 40°C durchgeführt, bevorzugterweise zwischen 20°C und 35 °C, besonders bevorzugt zwischen 25°C und 30°C, ganz besonders bevorzugt zwischen 27°C und 29°C und insbesondere bei 28°C.

In einer weiteren Ausführungsform der vorliegenden Erfindung umfasst das Niedrigsalzmedium weniger als 1,5 g/L Gesamtsalze.

In einer weiteren bevorzugten Ausführungsform der vorliegenden Erfindung entspricht der Gesamtsalzgehalt des Niedrigsalzmediums einem Wert < 15% des Salzgehaltes von Meerwasser, bevorzugt < 12% und besonders bevorzugt von < 10 %. Ganz besonders bevorzugt ist ein Gesamtsalzgehalt von < 8 % des Salzgehaltes von Meerwasser.

Es werden dem Niedrigsalzmedium keine Natriumsalze zugegeben. Dem erfindungsgemäßen Niedrigsalzmedium werden weiterhin keine Chloridsalze zugegeben.

Unter Zugabe wird erfindungsgemäß eine Zugabe sowohl in gelöster als auch in fester Form verstanden. Beispielsweise wäre das Zusatz von Meerwasser, auch in geringsten Mengen, erfindungsgemäß eine Zugabe von Natrium- bzw. Chloridsalzen. Auch der Zusatz von unüblichen Medienbestandteilen zum erfindungsgemäßen Medium, muß, falls diese unüblichen Medienbestandteile entsprechende Natrium- oder Chlorid-Ionen enthalten, als Zusatz dieser Salze verstanden werden. Es ist dem Fachmann jedoch klar, dass die üblichen (und zumeist notwendigen, d.h. unabdingbaren) Medienbestandteile Wasser (Leitungswasser), Hefeextrakt, Maisquellwasser u. ä. einen sehr geringen, nicht zu vermeidenden Eigenanteil an Natrium und Chlorid aufweisen. Der Zusatz solcher üblicher Medienbestandteile wird daher erfindungsgemäß nicht als Zugabe von Natrium- bzw. Chloridsalzen verstanden.

Hefeextrakt beispielsweise enthält unter 2 Gew.-% NaCl. Werden dem Medium daher in üblichem Maße Hefeextrakt zugesetzt, d.h. zwischen 10 und 20 g/L, erhöht sich der NaCl-Gehalt um weniger als 0,2 g/L. Dies wird erfindungsgemäß nicht als Zusatz von NaCl verstanden.

In einer besonders bevorzugten Ausführungsform ist dieses Medium daher frei von Natrium-und/oder Chloridsalz Zusätzen.

Ganz besonders bevorzugt liegt der Gesamtnatriumgehalt des Niedrigsalzmediums unter 2 g/L, bevorzugt unter 500 mg/L und ganz besonders bevorzugt unter 150 mg/L. Der Gesamtchloridgehalt des Niedrigsalzmediums liegt bevorzugterweise unter 2 g/L, bevorzugt unter 500 mg/L und ganz besonders bevorzugt unter 250 mg/L.

Besonders bevorzugt liegt die Summe der Gewichtsanteile an Na- und Cl-Ionen unter 1,75 g/L.

Das Niedrigsalzmedium umfasst ferner bevorzugterweise eine oder mehrere Kohlenstoffquellen, sowie eine oder mehrere Stickstoffquellen. Dem Fachmann sind für die Kultivierung von Mikroorganismen der Ordnung *Thraustochytriales* als Kohlenstoff- und Stickstoffquellen verwendbare Substanzen gut bekannt.

Verwendbare Kohlenstoffquellen sind beispielsweise Kohlenhydrate wie Glukose, Fruktose Xylose, Saccharose, Maltose, lösliche Stärke, Fucose, Glucosamin, Dextran, Glutaminsäure, Melasse, Glyzerin oder Mannitol oder auch Fette und Öle oder pflanzliche Hydrolysate.

Einsetzbare natürliche Stickstoffquellen sind beispielsweise Pepton, Hefeextrakt, Malzextrakt, Fleischextrakt, Casaminosäuren, Maisquellwasser oder Sojabohnen, einsetzbare organische Stickstoffquellen sind beispielsweise Glutamat und Harnstoff, aber auch anorganische Stickstoffquellen wie zum Beispiel Ammoniumacetat, Ammoniumhydrogencarbonat, Ammoniumsulfat oder Ammoniumnitrat können als Stickstoffquelle verwendet werden.

Das Niedrigsalzmedium kann alle weiteren dem Fachmann als der Kultivierung von Mikroorganismen der Ordnung *Thraustochytriales* förderliche Bestandteile enthalten, insbesondere anorganische Salze von beispielsweise Ca, Mg, K, Fe, Ni, Co, Cu, Mn, Mo oder Zn. Beispielhaft seien Phosphate wie Kaliumdihydrogenphosphat oder Carbonate wie Calciumcarbonat, Sulfate, wie Ammoniumsulfat, Magnesiumsulfat, Eisensulfat oder Kupfersulfat genannt. Weitere verwendbare anorganische Salze sind beispielsweise Halogenide, wie Kaliumbromid oder Kaliumjodid.

Ggf. kann das Medium zusätzliche Makro- oder Mikronährstoffe umfassen, wie Aminosäuren, Purine, Pyrimidine, Corn Steep Liquor, Proteinhydrolysate, Vitamine (wasserlöslich und/oder wasserunlöslich) und andere dem Fachmann gut bekannte Medienbestandteile. Antischaummittel können zugegeben werden, falls notwendig. Das Medium kann komplexe Bestandteile enthalten oder chemisch definiert sein.

Die Menge der einzelnen Komponenten kann variieren, solange kein negativer Effekt auf das Wachstum oder die Produktivität der Mikroorganismen vorliegt. Der Fachmann kann die Zusammensetzung entsprechend den Bedürfnissen des Mikroorganismus im Einzelfall leicht ermitteln. Im Allgemeinen wird die Kohlenstoffquelle in einer Konzentration von 50 bis 300 g/L und die Stickstoffquelle in einer Konzentration von 1 bis 30 g/L zugegeben. Vorzugsweise wird der Stickstoffgehalt in Abhängigkeit vom Kohlenstoffgehalt des Mediums gestellt.

Ein besonders bevorzugtes Niedrigsalzmedium umfasst gegebenenfalls neben weiteren Bestandteilen wie beispielsweise nutritiven Bestandteilen mindestens ein Salz ausgewählt aus der Gruppe bestehend aus Magnesiumsulfat, Calciumcarbonat und Kaliumphosphat, wobei das oder die Salz(e) bevorzugterweise zu je höchstens 3 g/L zugegeben werden, besonders bevorzugt zu je höchstens 1 g/L, ohne dass der erfindungsgemäße Gesamtsalzgehalt überschritten wird. Es ist besonders bevorzugt, wenn Magnesiumsulfat, Calciumcarbonat und Kaliumphosphat dem Medium zugegeben werden.

Bevorzugte nutritive Bestandteile sind Glukose, Hefeextrakt und/oder Maisquellwasser (Com Steep Liqour [CSL]) in den üblichen Mengen sowie weitere dem Fachmann geläufige nutritive Bestandteile.

Der pH-Wert des Mediums wird auf einen Bereich von 3 bis 10, bevorzugt 4 bis 8, besonders bevorzugt 5 bis 7 und ganz besonders bevorzugt 6 durch Zugabe einer entsprechenden Säure oder Lauge eingestellt.

Anschließend wird das Medium sterilisiert. Techniken zur Sterilisation von Medien sind dem Fachmann gut bekannt, beispielhaft seien Autoklavieren und Sterilfiltration genannt.

Die Kultivierung kann in Batch-, Fed-Batch- oder in kontinuierlicher Weise erfolgen, wie es dem Fachmann allgemein bekannt ist.

Eine Batch- oder Fed-Batch-Kultivierung erfolgt üblicherweise über 1 bis 12 Tage, bevorzugt 2-10 Tage, besonders bevorzugt für 3-9 Tage.

Die Medienbestandteile können dem Niedrigsalzmedium einzeln oder vermischt zugegeben werden, auch eine vorgefertigte Mischung ist denkbar. Die Bestandteile, insbesondere die Kohlenstoff- und Stickstoffquelle(n) oder bestimmte Mediumszusätze können vor oder während der Kultivierung zugegeben werden. Die Zugabe kann einmal oder mehrmals wiederholt oder auch kontinuierlich erfolgen.

Die produzierten PUFA liegen im Allgemeinen in Form von Neutralfetten zum Beispiel als Triacylglyzeride oder polare Lipide wie zum Beispiel Phosphatidylcholin, Phosphatidylethanolamin oder Phosphatidylinositol vor.

Allerdings werden für die Zwecke der vorliegenden Erfindung unter den Begriffen PUFA, n3-Fettsäure oder n3-Wirkstoffe alle möglichen Formen verstanden in denen die entsprechenden Fettsäuren vorliegen können, d. h. sowohl als freie Fettsäuren, Ester, Triglyceride, Phospholipide oder andere Derivate. All diese Substanzen werden im Folgenden zusammengefasst und die Begriffe werden synonym verwendet. Im weiteren können die PUFAs durch chemische oder biokatalytische Umesterung beispielsweise mit Hilfe geeigneter Enzyme (Lipasen) umgesetzt und angereichert werden, vor oder nach der Isolierung aus der Kultur.

Die Isolierung von PUFAs aus den fermentierten Mikroorganismen bzw. dem Medium und die Analyse des Fettsäurespektrums erfolgt nach für den Fachmann bekannten und üblichen Verfahren [Wanasundara,U.N., Wanasundara, J., Shahidi, F., Omega-3 fatty acid concentrates: a review of production technologies, Seafoods - Quality, Technology and Nutraceutical Applications, 2002, S. 157-174].

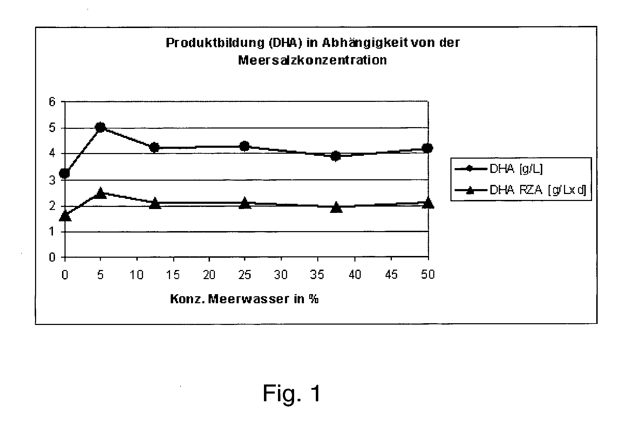

Figur 1 zeigt die Produktbildung von DHA in Abhängigkeit von der Salzkonzentration. Deutlich ist das Maximum im erfindungsgemäßen Bereich zu erkennen (Daten aus Beispiel 1)

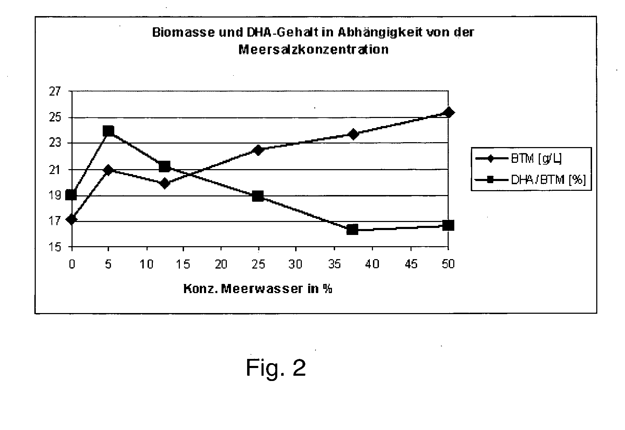

Figur 2 zeigt Biomasse und DHA-Gehalt in Abhängigkeit von der Salzkonzentration. Auch hier ist das Maximum im erfindungsgemäßen Bereich deutlich zu erkennen (Daten aus Beispiel 1).

Nachfolgend wird das dem erfindungsgemäßen Verfahren zugrunde liegende Fermentationsmedium anhand einiger Beispiele beschrieben. Das Fermentationsmedium sowie die Erfindung sind jedoch nicht auf diese Beispiele beschränkt.

### Beispiel 1: Einfluss unterschiedlicher Salzmengen im Medium auf die Produktion von PUFA durch Ulkenia sp. SAM 2179.

Stamm SAM 2179 (Ulkenia spec BP-5601; WO9803671) wurde in 300 ml Erlenmeyerkolben mit einer Schikane in 50 ml Medium kultiviert (Temperatur: 28°C, Schüttelrate: 150 rpm).

| Medium 1: DH1-Medium | |
|---|---|
| Glukose-Monohydrat (g/L): | 56,25 |
| Hefeextrakt (g/L): | 12,5 [Difco] |
| Tropic Marin (g/L): | 16,65 [Dr. Biener GmbH, Wartenberg, Germany] |

| | |
|---|---|
| pH-Wert mit HCl auf 6,0 eingestellt | |

| Medium 2: DH2-Medium (ohne Salz) | |
|---|---|
| Glukose-Monohydrat (g/L): | 56,25 |
| Hefeextrakt (g/L): | 12,5 [Difco] |

| | |
|---|---|
| pH-Wert mit HCl auf 6,0 eingestellt | |

Die Salze von Medium 1 (Tropic Marin) wurden in folgenden Konzentrationen eingesetzt: 1X (Medium 1), 0,75X (Medium 1.1), 0,5X (Medium 1.2), 0,25X (Medium 1.3) und 0,1X (Medium 1.4).

Zellernte erfolgte nach 48 h Kultivierung durch Zentrifugation. Anschließend wurden die Zellen gefriergetrocknet und die Biotrockenmasse bestimmt. Aufschluss der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert (Wanasundara,U.N., Wanasundara, J., Shahidi, F., Omega-3 fatty acid concentrates: a review of production technologies, Seafoods - Quality, Technology and Nutraceutical Applications, 2002, S. 157-174).

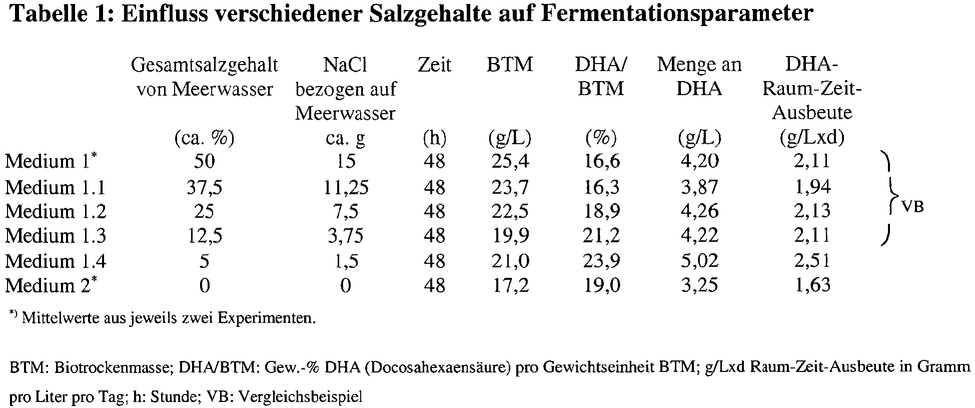

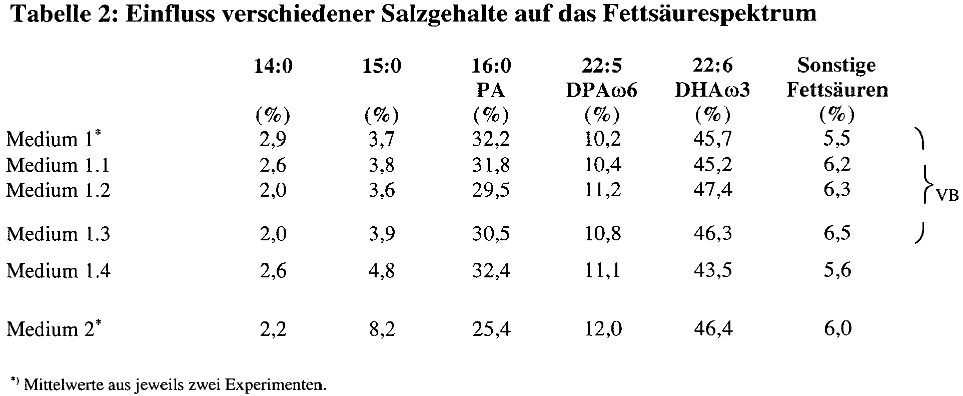

14:0 Myristinsäure; 15:0 Pentadekansäure; 16:0 Palmitinsäure; DPAω6: Docosapentaensäure (omega6); DHAω3: Docosahexaensäure (omega3)

Die Fermentation von *Ulkenia* sp. Stamm SAM 2179 bei unterschiedlichen Konzentrationen von Tropic Marin, ausgehend von etwa 50% bis 0% Meerwassersalzgehalt, zeigt tendenziös eine Abnahme der Biomasse mit abnehmendem Salzgehalt im Medium. Allerdings bildet die Fermentation bei einem niedrigen Salzgehalt von etwa 5% des Meerwassers überraschenderweise eine maßgebliche Ausnahme. Hier zeigt der Verlauf der Biomasseproduktion erstaunlicherweise ein Zwischenmaximum, bei dem wieder höhere Werte erzielt werden. Außerdem nimmt der Anteil der vorherrschenden Fettsäure DHA pro Biotrockenmasse mit abnehmender Salzkonzentration zu und hat seinen höchsten Wert ebenfalls bei 5% des Meerwassersalzgehaltes, wobei er bei noch niedrigerem Salzgehalt wieder abnimmt. Daraus ergibt sich für die Raum-Zeit-Ausbeute der essentiellen PUFA DHA ein Maximum, bei etwa 5% Meerwassersalzgehalt (siehe Tabelle 1). Dieses Maximum führt zu einer Steigerung der DHA-Raum-Zeit-Ausbeute von mehr als 15%. Bezogen auf das gesamte Fettsäurespektrum liegt dabei der Anteil von DHA bei etwa 5% Meersalzgehalt zwar etwas niedriger als bei höheren bzw. niedrigeren Salzgehalten (siehe Tabelle 2), die Gesamtproduktivität an DHA und generell Fettsäuren, bzw. Öl ist jedoch gerade hier am größten (siehe Tabelle 1). Basierend auf diesen überraschenden Ergebnissen wurde das der Erfindung zugrunde liegende Niedrigsalzmedium entwickelt.

### Beispiel 2: Produktion von PUFA durch Ulkenia sp. SAM 2179 in verschiedenen Fermentationsmedien.

Stamm SAM 2179 wurde in 300 ml Erlenmeyerkolben mit einer Schikane in 50 ml Medium kultiviert (Temperatur: 28°C, Schüttelrate: 150 rpm).

| Medium 1: DH1-Medium | |
|---|---|
| Glukose-Monohydrat (g/L): | 56,25 |
| Hefeextrakt (g/L): | 12,5 [Difco] |
| Tropic Marin (g/L): | 16,65 [Dr. Biener GmbH, Wartenberg, Germany] |

| | |
|---|---|
| pH-Wert mit HCl auf 6,0 eingestellt | |

| Medium 2: DH2-Medium (ohne Salz): | |
|---|---|
| Glukose-Monohydrat (g/L): | 56,25 |
| Hefeextrakt (g/L): | 12,5 [Difco] |

| | |
|---|---|
| pH-Wert mit HCl auf 6,0 eingestellt | |

| Medium 3: DH3-Medium (mit Salzsupplement ohne Natrium- und ohne Chlorid-Zusatz): | | |
|---|---|---|
| Glukose-Monohydrat (g/L): | 56,25 | |
| Hefeextrakt (g/L): | 12,5 | [Difco] |
| Magnesiumsulfat (g/L): | 1 | |
| Calciumcarbonat (g/L) | 1 | |
| Kaliumphosphat (g/L) | 1 | |

| | | |
|---|---|---|
| pH-Wert mit H₂SO₄ auf 6,0 eingestellt | | |

Zellernte erfolgte nach 48 h Kultivierung durch Zentrifugation. Anschließend wurden die Zellen gefriergetrocknet und die Biotrockenmasse bestimmt. Aufschluss der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 3: Einfluss verschiedener Salzgehalte auf Fermentationsparameter**

| | Gesamt-salzgehalt von Meerwasser | NaCl bezogen auf Meerwasser | Zeit | BTM | DHA/ BTM | Menge an DHA | DHA-Raum-Zeit-Ausbeute | |
|---|---|---|---|---|---|---|---|---|
| | ca. % | ca. g | (h) | (g/L) | (%) | (g/L) | (g/Lxd) | |
| Medium 1* | 50 | 15 | 48 | 25,4 | 16,6 | 4,20 | 2,11 | Vergleichsbeispiel |
| Medium 2* | 0 | 0 | 48 | 17,2 | 19,0 | 3,25 | 1,63 | |
| Medium 3** | 10 | 0 | 48 | 22,7 | 18,9 | 4,29 | 2,14 | |

| | | | | | | | | |
|---|---|---|---|---|---|---|---|---|
| *) Mittelwerte aus jeweils zwei Experimenten. ^{**)} Mittelwerte aus jeweils drei Experimenten. | | | | | | | | |

Das erfindungsgemäße Niedrigsalzmedium ohne Natrium- und ohne Chlorid-Zusatz wurde zunächst in einer Konzentration von etwa 10% des Meerwassersalzgehaltes in der Fermentation eingesetzt (Medium 3). Die dabei erzielte Biomassen sind dabei im Vergleich zu Fermentationen mit etwa 50% Meerwassersalzgehalt etwas niedriger, der Gehalt an DHA pro Biotrockenmasse ist jedoch größer und führt so überraschenderweise zu insgesamt gleichen, bzw. sogar erhöhten Raum-Zeit-Ausbeuten (siehe Tabelle 3). Dies ist von wesentlichem Vorteil für eine spätere Aufarbeitung der Biomasse zur Gewinnung von DHA. Überraschenderweise zeigte sich also, dass für die Fermentation auf den Zusatz von Natrium und/oder Chlorid (den Hauptsalzen von Meerwasser) mit Vorteil völlig verzichtet werden kann.

### Beispiel 3: Einfluss unterschiedlicher Salzmengen im Medium ohne Natrium- und ohne Chloridsalz-Zusatz auf die Produktion von PUFA durch Ulkenia sp. SAM 2179.

Stamm SAM 2179 wurde in 300 ml Erlenmeyerkolben mit einer Schikane in 50 ml Medium kultiviert (Temperatur: 28°C, Schüttelrate: 150 rpm).

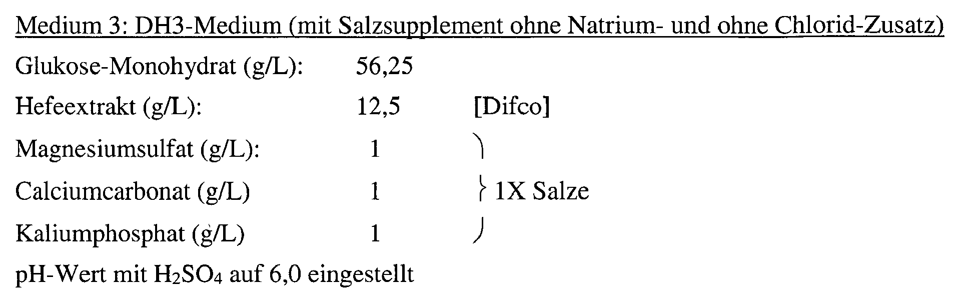

Die Salze von Medium 3 wurden in folgenden Konzentrationen eingesetzt: 10X mit je 10g/L, 2X mit je 2g/L, 1X mit je 1g/L, 0,5X mit je 0,5g/L oder 0,25X mit je 0,25g/L.

Zellernte erfolgte nach 48h Kultivierung durch Zentrifugation. Anschließend wurden die Zellen gefriergetrocknet und die Biotrockenmasse bestimmt. Aufschluss der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 4: Einfluss verschiedener Salzgehalte auf Fermentationsparameter**

| | Gesamtsalzgehalt von Meerwasser | NaCl bezogen auf Meerwasser | Zeit | BTM | DHA/ BTM | Menge an DHA | DHA-Raum-Zeit-Ausbeute | |
|---|---|---|---|---|---|---|---|---|
| | ca. % | ca. g | (h) | (g/L) | (%) | (g/L) | (g/Lxd) | |
| Medium 3 (10X) | 100 | 0 | 48 | 32,5 | 12,5 | 4,05 | 2,03 | Vergleichs -beispiel |
| Medium 3 (2X) | 20 | 0 | 48 | 23,8 | 20,3 | 4,82 | 2,41 | |
| Medium 3 (1X)** | 10 | 0 | 48 | 22,7 | 18,9 | 4,29 | 2,14 | |
| Medium 3 (0,5X) | 5 | 0 | 48 | 22,7 | 21,2 | 4,82 | 2,41 | |
| Medium 3 (0,25X) | 2,5 | 0 | 48 | 21,2 | 19,7 | 4,18 | 2,09 | |

| | | | | | | | | |
|---|---|---|---|---|---|---|---|---|
| ^{**)} Mittelwerte aus jeweils drei Experimenten. | | | | | | | | |

Zur Bestimmung der optimalen Salzkonzentration des Fermentationsmediums ohne Natrium- und Chlorid-Zusatz wurde der Mikroorganismus *Ulkenia* spec. Stamm 2179 in dem oben genannten Medium mit verschiedenen Salzkonzentrationen fermentiert. Auch in diesem Fall ist der DHA-Gehalt pro Biotrockenmasse bei einem Salzgehalt von 5% des Meerwassers am höchsten (siehe auch Beispiel 1). Die Produktivität als Raum-Zeit-Ausbeute ausgedrückt liegt bei diesem Salzgehalt ebenfalls bei einem optimalen Wert (siehe Tabelle 4).

### Beispiel 4: Produktion von PUFA durch Schizochytrium Stamm SR21 (Schizochytrium spec., MYA-1381; EP0823475) in verschiedenen Fermentationsmedien.

*Schizochytrium* Stamm SR21 wurde in 300 ml Erlenmeyerkolben mit einer Schikane in 50 ml Medium kultiviert (Temperatur: 28°C, Schüttelrate:150 rpm).

| Medium 1: DH1-Medium | | |
|---|---|---|
| Glukose-Monohydrat (g/L): | 56,25 | |
| Hefeextrakt (g/L): | 12,5 | [Difco] |
| Tropic Marin (g/L): | 16,65 | [Dr. Biener GmbH, Wartenberg, Germany] |

| | | |
|---|---|---|
| pH-Wert mit HCl auf 6,0 eingestellt | | |

| Medium 2: DH2-Medium (ohne Salz) | | |
|---|---|---|
| Glukose-Monohydrat (g/L): | 56,25 | |
| Hefeextrakt (g/L): | 12,5 | [Difco] |

| | | |
|---|---|---|
| pH-Wert mit HCl auf 6,0 eingestellt | | |

| Medium 3: DH3-Medium (mit Salzsupplement ohne Natrium- und ohne Chlorid-Zusatz) | | |
|---|---|---|
| Glukose-Monohydrat (g/L): | 56,25 | |
| Hefeextrakt (g/L): | 12,5 | [Difco] |
| Magnesiumsulfat (g/L): | 1 | |
| Calciumcarbonat (g/L) | 1 | |
| Kaliumphosphat (g/L) | 1 | |

| | | |
|---|---|---|
| pH-Wert mit H₂SO₄ auf 6,0 eingestellt | | |

Zellernte erfolgte nach 48h Kultivierung durch Zentrifugation. Anschließend wurden die Zellen gefriergetrocknet und die Biotrockenmasse bestimmt. Aufschluss der Zellen und Bestimmung der Fettsäuren erfolgte durch Hitzebehandlung für 2 Stunden in 10%iger methanolischer Salzsäure bei 60°C (unter Rühren). Die Ester wurden dann im Gaschromatographen zur Bestimmung der Fettsäurezusammensetzung analysiert.

**Tabelle 5: Einfluss verschiedener Salzgehalte auf Fermentationsparameter**

| | Gesamtsalzgehalt von Meerwasser | NaCl bezogen auf Meerwasser | Zeit | BTM | DHA/ BTM | Menge an DHA | DHA-Raum-Zeit-Ausbeute | |
|---|---|---|---|---|---|---|---|---|
| | ca. % | ca. g | (h) | (g/L) | (%) | (g/L) | (g/Lxd) | |
| Medium 1 SR 21 | 50 | 15 | 48 | 27,1 | 13,7 | 3,61 | 1,81 | Vergleichsbeispiel |
| Medium 2 SR 21 | 0 | 0 | 48 | 19,5 | 18,2 | 3,56 | 1,78 | |
| Medium 3 SR 21 | 10 | 0 | 48 | 22,3 | 18,7 | 4,17 | 2,09 | |

**Tabelle 6: Einfluss verschiedener Salzgehalte auf das Fettsäurespektrum**

| | **14:0** | **15:0** | **16:0 PA** | **22:5 DPAω6** | **22:6 DHAω3** | **Sonstige Fettsäuren** | |
|---|---|---|---|---|---|---|---|
| | (%) | (%) | (%) | (%) | (%) | (%) | |
| Medium 1 SR 21 | 3,5 | 3,3 | 43,4 | 7,5 | 37,5 | 4,8 | Vergleichsbeispiel |
| Medium 2 SR 21 | 3,5 | 5,3 | 43,1 | 7,4 | 36,5 | 4,2 | |
| Medium 3 SR 21 | 3,5 | 4,1 | 44,4 | 7,1 | 36,1 | 4,8 | |

Das in der Erfindung beschriebene Niedrigsalzmedium führt auch bei anderen Organismen der *LabyrinthuLomycota* zu einer Optimierung der Produktion von PUFA. So kann beispielsweise der Mikroorganismus *Schizochytrium* spec. Stamm SR21 in Niedrigsalzmedium ohne Natrium- und ohne Chlorid-Zusatz fermentiert werden. Der DHA-Gehalt bezogen auf die Trockenmasse besitzt in diesem Fall bei einem Salzgehalt von 10% des Meerwassers ebenfalls ein Optimum. Darüber hinaus zeigt sich hier ein noch stärkerer Effekt auf die Raum-Zeit-Ausbeute von DHA und damit auf die Produktivität der Fermentation (siehe Tabelle 5). Der DHA-Gehalt bezogen auf das gesamte Fettsäurespektrum ist auch in diesem Fall etwas erniedrigt (siehe Tabelle 6), was aber einer überraschend hohen Raum-Zeit-Ausbeute an DHA (siehe Tabelle 5) nicht schadet. Das der Erfindung zugrunde liegende optimierte Niedrigsalzmedium führt zu einer generellen Produktionssteigerung von PUFAs in verschiedenen Mitgliedern der *Labyrinthulomycota.*

## Patentansprüche

1. Öl mit einem Gehalt von mindestens 10% DHA (Docohexanoic acid), erhältlich durch ein Verfahren zur Gewinnung von DHA-haltigen Ölen aus Mikroorganismen der Ordnung Thraustochytriales, die eine Produktion von mehr als 10 % DHA, bevorzugt mehr als 14 %, mehr bevorzugt mehr als 18 % pro Biotrockenmasse zeigen, **dadurch gekennzeichnet, dass** man
a) die Mikroorganismen in einem Fermentationsmedium ohne Zusatz von Natriumsalzen und Chloridsalzen während der Dauer der Kultivierung bei einem Gesamtsalzgehalt von weniger als 3,5 g/l kultiviert, und die Summe der Gewichtsanteile an Na⁺- und Cl⁻-Ionen weniger als 1,75 mg/L umfasst,
b) aus den Mikroorganismen Biomasse und/oder Kulturbrühe gewinnt und
c) das in der Biomasse und/oder der Kulturbrühe enthaltene Öl isoliert.

2. Öl mit einem Gehalt von mindestens 10% DPA (Docopentanoic acid), erhältlich durch ein Verfahren zur Gewinnung von DHA-haltigen Ölen aus Mikroorganismen der Ordnung Thraustochytriales, die eine Produktion von mehr als 5 %, bevorzugt mehr als 7 % und mehr bevorzugt mehr als 10 % DPA pro Biotrockenmasse zeigen, **dadurch gekennzeichnet, dass** man
a) die Mikroorganismen in einem Fermentationsmedium ohne Zusatz von Natriumsalzen und Chloridsalzen während der Dauer der Kultivierung bei einem Gesamtsalzgehalt von weniger als 3,5 g/l kultiviert, und die Summe der Gewichtsanteile an Na⁺- und Cl⁻-Ionen weniger als 1,75 mg/L umfasst,
b) aus den Mikroorganismen Biomasse und/oder Kulturbrühe gewinnt und
c) das in der Biomasse und/oder der Kulturbrühe enthaltene Öl isoliert.

3. Öl nach Anspruch 1 oder 2, wobei im Herstellungsverfahren das in Schritt c) isolierte Öl eine Reinheit von mehr als 90 % aufweist.

4. Öl nach einem der vorstehenden Ansprüche, wobei im Herstellungsverfahren dem Fermentationsmedium bis zu 3g/L CaCO₃, bevorzugt bis zu 1g/L CaCO₃ zugesetzt werden.

5. Öl nach einem der vorstehenden Ansprüche, wobei im Herstellungsverfahren der Gesamtsalzgehalt des Fermentationsmediums im Bereich < 8 % des Salzgehaltes von Meerwasser liegt.

6. Öl nach einem der vorstehenden Ansprüche, **dadurch gekennzeichnet, dass** im Herstellungsverfahren der Gesamtnatriumgehalt des Fermentationsmediums unter 150 mg/L liegt.

7. Öl nach einem der vorstehenden Ansprüche, **dadurch gekennzeichnet, dass** im Herstellungsverfahren der Gesamtchloridgehalt des Fermentationsmediums unter 250 mg/L liegt.

8. Öl nach einem der vorstehenden Ansprüche, **dadurch gekennzeichnet, dass** im Herstellungsverfahren das Fermentationsmedium Glukose, Hefeextrakt, Magnesiumsulfat, Calciumcarbonat und Kaliumphosphat umfasst.

9. Öl nach einem der vorstehenden Ansprüche, **dadurch gekennzeichnet, dass** im Herstellungsverfahren das Fermentationsmedium Glukose, Maisquellwasser, Magnesiumsulfat, Calciumcarbonat und Kaliumphosphat umfasst.

10. Öl nach Anspruch 8 oder 9, **dadurch gekennzeichnet, dass** im Herstellungsverfahren das Fermentationsmedium Magnesiumsulfat, Calciumcarbonat und Kaliumphosphat zu je höchstens 3 g/L, besonders bevorzugt zu je höchstens 1 g/L umfasst.

11. Öl nach einem der vorstehenden Ansprüche, **dadurch gekennzeichnet, dass** im Herstellungsverfahren das Fermentationsmedium einen pH-Wert zwischen 3 und 10, bevorzugt zwischen 5 und 7 aufweist.

12. Öl nach einem der vorstehenden Ansprüche, **dadurch gekennzeichnet, dass** im Herstellungsverfahren die Kultivierung zwischen 10°C und 40°C, bevorzugt zwischen 25°C und 35°C erfolgt.

13. Öl nach einem der vorstehenden Ansprüche, **dadurch gekennzeichnet, dass** im Herstellungsverfahren die Kultivierung für 1 bis 10 Tage, bevorzugt für 3 bis 9 Tage erfolgt.

14. Öl nach einem der vorstehenden Ansprüche, **dadurch gekennzeichnet, dass** im Herstellungsverfahren der verwendete Mikroorganismus zur Gattung *Schizochytrium, Thraustochytrium* oder *Ulkenia* gehört.

15. Öl nach einem der vorstehenden Ansprüche, **dadurch gekennzeichnet, dass** im Herstellungsverfahren der Mikroorganismus *Ulkenia* spec. SAM 2179 oder *Schizochytrium* spec. SR 21 verwendet wird.

16. Futtermittel oder Nahrungsmittel umfassend Biomasse, erhältlich durch ein Verfahren zur Gewinnung von DHA-haltiger Biomasse aus Mikroorganismen der Ordnung Thraustochytriales, die eine Produktion von mehr als 10 % DHA, bevorzugt mehr als 14 %, mehr bevorzugt mehr als 18 % pro Biotrockenmasse zeigen, **dadurch gekennzeichnet, dass** man
a) die Mikroorganismen in einem Fermentationsmedium ohne Zusatz von Natriumsalzen und Chloridsalzen während der Dauer der Kultivierung bei einem Gesamtsalzgehalt von weniger als 3,5 g/l kultiviert, und die Summe der Gewichtsanteile an Na⁺- und Cl⁻-Ionen weniger als 1,75 mg/L umfasst, und
b) aus den Mikroorganismen Biomasse gewinnt.

17. Futtermittel oder Nahrungsmittel umfassend Biomasse, erhältlich durch ein Verfahren zur Gewinnung von DPA-haltiger Biomasse aus Mikroorganismen der Ordnung Thraustochytriales, die eine Produktion von mehr als 5 %, bevorzugt mehr als 7 % und mehr bevorzugt mehr als 10 % DPA pro Biotrockenmasse zeigen, **dadurch gekennzeichnet, dass** man
a) die Mikroorganismen in einem Fermentationsmedium ohne Zusatz von Natriumsalzen und Chloridsalzen während der Dauer der Kultivierung bei einem Gesamtsälzgehalt von weniger als 3,5 g/l kultiviert, und die Summe der Gewichtsanteile an Na⁺- und Cl⁻-Ionen weniger als 1,75 mg/L umfasst, und
b) aus den Mikroorganismen Biomasse gewinnt.
